# FASCICULE DE BREVET EUROPEEN

(11) **EP 4 018 191 B1**
(45) Date de publication et mention de la délivrance du brevet: **26.07.2023**
(21) Numéro de dépôt: 20753960.2
(22) Date de dépôt: 17.08.2020
(51) Int. Cl.: G01N 33/49, C12Q 1/04, G01N 27/403

(54) **SYSTÈME INCLUANT UN CAISSON ET UN CONTENEUR INSTRUMENTÉ POUR LA DÉTECTION DE PRÉSENCE DE MICRO-ORGANISMES DANS UN ÉCHANTILLON LIQUIDE**
SYSTEM MIT EINEM GEHÄUSE UND EINEM INSTRUMENTIERTEN BEHÄLTER ZUM NACHWEIS DER ANWESENHEIT VON MIKROORGANISMEN IN EINER FLÜSSIGEN PROBE
SYSTEM INCLUDING A HOUSING AND AN INSTRUMENTED CONTAINER FOR DETECTING THE PRESENCE OF MICRO-ORGANISMS IN A LIQUID SAMPLE

(30) Priorité: 22.08.2019 FR 1909333
(43) Date de publication de la demande: 29.06.2022
(73) Titulaire: Commissariat à l'Energie Atomique et aux Energies Alternatives, 75015 Paris (FR)
(72) Inventeur: GOUGIS, Maxime, 38054 GRENOBLE Cedex 9 (FR); MARCOUX, Pierre, 38054 GRENOBLE Cedex 09 (FR)
(74) Mandataire: INNOV-GROUP
(86) Numéro de dépôt international: PCT/EP2020/072947
(87) Numéro de publication internationale: WO 2021/032654

(56) Documents cités:
- US-A- 5 518 923
- US-A1- 2011 284 374
- US-A1- 2019 004 004
- Michelle ; Ablutz ET AL: "Cell Culture Monitoring with the Vi-CELL MetaFLEX", , 23 mai 2017 (2017-05-23), XP055693254, Extrait de l'Internet: URL:https://www.beckman.com/gated-media?me diaId={CCD8FEBC-91AE-4883-875C-C2C27FC5DBF 1} [extrait le 2020-05-08]
- S. H. LIM ET AL: "Colorimetric Sensor Array Allows Fast Detection and Simultaneous Identification of Sepsis-Causing Bacteria in Spiked Blood Culture", JOURNAL OF CLINICAL MICROBIOLOGY, vol. 52, no. 2, 11 décembre 2013 (2013-12-11), pages 592-598, XP055229593, US ISSN: 0095-1137, DOI: 10.1128/JCM.02377-13

## Description

### Domaine technique de l'invention

La présente invention se rapporte à un système comprenant notamment un caisson dans lequel est placé un conteneur instrumenté et à un procédé de détection de présence de micro-organismes dans un échantillon liquide placé dans le conteneur du système. La détection pourra notamment se faire par voie électrochimique.

### Etat de la technique

Le sang est en temps normal totalement stérile. Tout micro-organisme présent dans le système sanguin représente donc une menace vitale pour le corps humain. L'hémoculture est actuellement la seule méthode de diagnostic des infections sanguines. Il s'agit d'un test fréquent en microbiologie clinique. L'hémoculture consiste en premier lieu en la culture du sang dans un bouillon nutritif en condition aérobie et anaérobie, sous incubation à 37°C dans des automates adaptés à ce type d'échantillons, afin d'obtenir une croissance bactérienne. L'objectif consiste à amplifier la quantité de bactéries présentes dans l'échantillon, en lui fournissant des conditions favorables de croissance, et ainsi à détecter la présence de bactérie dans le sang chez un patient présentant un état septique. Cette amplification est d'autant plus nécessaire que la concentration bactérienne dans le sang au cours des bactériémies est toujours faible, de l'ordre d'une ou de quelques bactéries par millilitre de sang prélevé. Cette première étape de l'hémoculture consiste donc en une simple détection de présence, sans identification, à partir d'un échantillon sans flore normale : toute présence de bactérie ou champignon conduit donc à un test positif.

La note d'application Ablutz et al.: "Cell Culture Monitoring with the Vi-CELL MetaFLEX", 23 mai 2017, XP055693254, décrit l'emploi d'un système disponible sur le marché dans ce contexte.

Cette détection sera ensuite suivie d'une subculture sur milieu gélosé pour isoler le pathogène sous forme de colonies, permettant ensuite de procéder à l'identification du pathogène et à son antibiogramme. Dans le cas des infections sanguines, les examens sont urgents et le délai de rendu des résultats (positivité de l'hémoculture, identité du pathogène, antibiogramme) a un impact indéniable sur le devenir des patients (mortalité, durée d'hospitalisation, complications ...). Les résultats d'hémoculture permettent en effet d'adapter l'antibiothérapie au cas spécifique du patient : plus une antibiothérapie adaptée et efficace intervient précocement, plus les chances de survie du patient s'accroissent. Chaque heure de retard à l'instauration d'une antibiothérapie adaptée est associée à une augmentation de la mortalité.

Les industriels du diagnostic ont oeuvré pendant de nombreuses années à réduire le délai de positivité des hémocultures, ainsi qu'à réduire les temps d'analyse sur les tests d'identification et d'antibiogramme. Actuellement, deux grands types d'automates coexistent :
- Des appareils qui suivent la quantité d'acide carbonique généré dans la phase liquide, grâce à une matrice polymère (silicone) chargée avec un chromophore ou un fluorophore sensible au pH.
- Des appareils employés pour déceler une augmentation de la pression totale dans la phase gaz.

Ces deux technologies ont en commun d'être mises en oeuvre dans des automates de grande taille, avec l'impossibilité de démarrer le test tant que le flacon n'est pas dans l'automate (une pré-incubation génère des faux-négatifs), d'où la perte de précieuses heures avant la mise en place d'une antibiothérapie optimisée. Une étude en 2013 a montré que le temps de transport moyen était de 9 h (écart interquartile : 3-15 h), avec 6% de flacons présentant un temps de transport supérieur à 20 h.

Il existe donc un besoin de disposer d'une solution qui :
- permette de réduire la durée entre le prélèvement de l'échantillon et le démarrage de l'analyse de l'échantillon prélevé ;
- soit facilement transportable pour disposer d'une solution disponible sur le terrain ;
- soit facile à mettre en oeuvre, même par du personnel peu qualifié ;

### Exposé de l'invention

Ce besoin est comblé par un système de détection de présence de micro-organismes présent dans un échantillon, ce système comportant :
- Un caisson présentant un espace interne fermé délimité par un réceptacle et un couvercle destiné à être apposé sur le réceptacle pour fermer le caisson de manière hermétique,
- Une unité électronique d'acquisition de données de mesures de pH qui est intégrée audit caisson,
- Deux deuxièmes contacts électriques reliés à l'unité électronique d'acquisition et agencés à l'intérieur du caisson,
- Une unité de chauffage intégrée au caisson pour chauffer l'espace interne du caisson,
- Une unité de mesure et de gestion de la température comprenant une sonde de mesure de température placée à l'intérieur du caisson,
- Une source d'alimentation électrique pour alimenter l'unité électronique d'acquisition et l'unité de chauffage,
- Une unité de traitement reliée à ladite unité électronique d'acquisition et configurée pour traiter les données de mesure et comportant un module de détection de présence de micro-organismes par analyse de la variation du pH mesuré dans l'échantillon liquide,
- Un conteneur instrumenté logé dans l'espace interne du réceptacle et destiné à recevoir ledit échantillon, ledit conteneur comprenant :
   - Des parois comportant une première surface dite interne, en contact avec son volume interne destiné à recevoir ledit échantillon et une surface dite externe opposée à ladite surface interne,
   - Une électrode de référence et une électrode de mesure de pH intégrées à au moins une première paroi desdites parois du conteneur et comprenant chacune au moins deux parties conductrices reliées entre elles, une première partie agencée sur la surface interne et destinée à être en contact avec l'échantillon liquide placé à l'intérieur du conteneur et une deuxième partie agencée sur la surface externe et comprenant deux premiers contacts électriques distincts accessibles à l'extérieur du conteneur, ses premiers contacts électriques étant en appui chacun contre un deuxième contact électrique distinct dudit caisson.

Selon une particularité, les deuxièmes contacts électriques sont réalisés sous la forme de deux anneaux conducteurs agencés de manière concentrique.

Selon une autre particularité, l'unité électronique d'acquisition comporte un premier module de communication sans-fil et l'unité de traitement comporte un deuxième module de communication sans-fil relié au premier module de communication sans-fil via une liaison de communication sans-fil.

Selon une autre particularité, le système comporte une unité d'agitation par vibration intégrée audit réceptacle.

Selon une autre particularité, l'unité de traitement est intégrée audit caisson.

Selon une autre particularité, la première partie de l'électrode de référence et celle de l'électrode de mesure sont réalisées chacune sous la forme d'une pastille affleurante à la surface interne de ladite première paroi.

Selon une autre particularité, les deux pastilles sont réalisées chacune sous la forme d'un dépôt d'une encre conductrice sérigraphiée sur la surface interne de ladite première paroi.

Selon une autre particularité, la pastille de l'électrode de mesure est réalisée dans un matériau choisi parmi l'oxyde d'iridium et un polymère conducteur.

Selon une autre particularité, la pastille de l'électrode de référence est de type Ag/AgCI.

Selon une autre particularité, les deux premiers contacts électriques sont intégrés à ladite première paroi de manière à former chacun un plot de contact ou deux anneaux concentriques.

L'invention concerne également un procédé de détection de présence micro-organismes dans un échantillon liquide mis en oeuvre à l'aide du système tel que défini ci-dessus, ledit procédé comportant les étapes suivantes :
Introduction de l'échantillon liquide et d'un milieu de culture dans le conteneur pour obtenir un mélange liquide,
Introduction dudit conteneur dans l'espace interne du caisson,
Réglage de la température d'incubation dans l'espace interne du caisson à une valeur fixe adaptée au milieu de culture,
Mesure du pH au cours du temps dans le mélange liquide présent dans le conteneur pour obtenir une courbe de variation du pH en fonction du temps,
Détermination de la présence ou de l'absence de micro-organismes par analyse de ladite courbe de variation du pH en fonction du temps.

Selon une particularité, le procédé comporte une étape d'agitation du conteneur pour mélanger l'échantillon liquide et le milieu de culture.

Selon une autre particularité, le procédé comporte une étape de réglage de la durée d'incubation.

La solution de l'invention permet ainsi de doter chaque conteneur de son propre instrument de suivi de la croissance microbienne, ce qui permet de démarrer le test d'hémoculture à l'extérieur du laboratoire d'analyse, sur le lieu même de prélèvement.

### Brève description des figures

D'autres caractéristiques et avantages vont apparaître dans la description détaillée qui suit faite en regard des dessins annexés dans lesquels :
- La figure 1A représente un conteneur instrumenté de type flacon conforme à celui employé dans le système de l'invention.
- La figure 1B représente en vue de dessus, le fond dudit flacon et montre les deux électrodes intégrées au flacon.
- La figure 2A représente, de manière schématique, le système de détection de présence de micro-organismes conforme à l'invention.
- La figure 2B représente en vue de dessus le principe de réalisation des contacts électriques employés dans le caisson du système de l'invention.
- La figure 3 représente un diagramme présentant les différentes étapes du procédé de l'invention mis en oeuvre à l'aide du système de la figure 2.
- La figure 4 représente deux courbes de variation du pH en fonction du temps, obtenues grâce au système conforme à l'invention.

### Description détaillée d'au moins un mode de réalisation

Dans la suite de la description, le terme "intégrer" est à comprendre comme faisant partie d'un même ensemble, non séparable sans l'emploi d'outils adaptés.

L'invention proposée permet notamment de réduire considérablement la durée entre le prélèvement d'un échantillon et son analyse.

L'invention consiste entre autres à réaliser un automate autonome et individualisé pour effectuer une détection de présence de micro-organismes dans un échantillon liquide, par mesure électrochimique de pH.

De manière particulière, le système sera adapté à la détection de présence d'un pathogène dans un échantillon de sang, par mesure électrochimique de pH lors d'une hémoculture. Cependant il pourra également être adapté à d'autres liquides biologiques, tels que par exemple des liquides articulaires, pleuraux, péricardiques et autres liquides de ponction, liquides vitrés et des pus.

Dans le domaine clinique également, il pourra servir au test de stérilité d'objets solides tels que des biopsies. Au niveau industriel, un tel système permet les tests de stérilité sur des produits finis (flaconnages, seringues, aiguilles, prothèses, poches de nutrition parentérale, lentilles de contact, médicaments injectables, *etc*.), ou des matières premières (principes actifs, excipients, produits cosmétiques, *etc*.). Il s'agit alors d'immerger l'objet à tester dans un milieu nutritif gélosé ou liquide, puis de suivre lors de l'incubation (à 30°C ou 37°C) si une croissance microbienne intervient dans le milieu nutritif. Lorsque le test a lieu avec un milieu nutritif gélosé, il est alors plutôt réalisé dans une boite de Pétri, auquel cas le système est adapté non pas sur un flacon mais dans une boite de Pétri.

La détection devant être non spécifique, il est important de la baser sur un indicateur particulièrement générique : l'émission de CO₂ est l'un de ces paramètres puisque ce métabolite est commun à tous les micro-organismes. Le métabolisme élevé des bactéries (de l'ordre du W/g) s'accompagne en effet d'une forte émission de CO₂, de l'ordre de 700 000 molécules par seconde et par bactérie lors de la phase exponentielle de la croissance. C'est ce phénomène que le système de l'invention permet de mettre à profit, en détectant l'acidification consécutive à l'émission de CO₂. Dans le cas d'un échantillon de sang, l'invention vise donc notamment à pouvoir détecter un pathogène dans une hémoculture grâce à des électrodes assurant le suivi électrochimique du pH. L'électrochimie permet d'assurer un suivi de l'hémoculture avec un capteur bas coût, compact, jetable, stérilisable, peu gourmand en énergie et dont la production peut être implémentée à grande échelle. De même, dans le cas des tests de stérilité, l'invention vise à détecter l'acidification du milieu nutritif liquide ou gélosé consécutive à la croissance microbienne si l'objet testé n'est pas stérile.

Ce principe est mis en oeuvre en employant notamment un conteneur pouvant être par exemple un flacon 1 instrumenté destiné à recevoir l'échantillon ECH liquide prélevé ou une boîte de Pétri comme évoqué ci-dessus.

Dans la suite de la description, l'invention est décrite pour un flacon 1 mais pourra s'adapter à tous types de conteneur.

Le flacon est réalisé sous la forme d'un élément monobloc, avantageusement en matériau plastique jetable et éventuellement biosourcé.

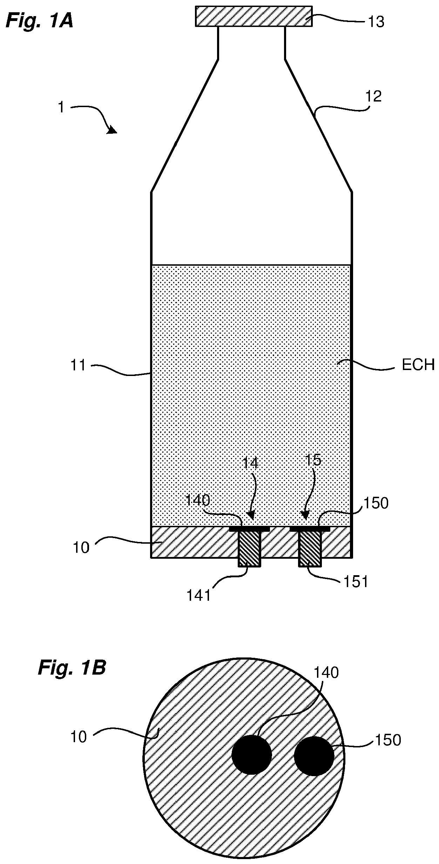

En référence à la figure 1A, ce flacon 1 peut présenter une forme classique dotée d'une paroi inférieure 10 formant le cul du flacon, d'une paroi latérale 11 formant le corps et d'un col 12 dans sa partie supérieure, le col se terminant par le goulot. Les parois du flacon délimitent le volume interne du flacon. Les parois du flacon présentent une surface interne située à l'intérieur du flacon et une surface externe située à l'extérieur du flacon. Le flacon comporte également un bouchon 13 apposé sur son goulot pour le fermer de manière hermétique.

Selon une particularité de l'invention, le flacon 1 intègre deux électrodes 14, 15, une électrode de mesure 14 et une électrode de référence 15. Les deux électrodes sont avantageusement intégrées dans la paroi inférieure 10 du flacon 1. Les électrodes 14, 15 peuvent être recouvertes d'une couche de protection afin d'améliorer la stabilité dans le temps du flacon et notamment pour protéger les électrodes d'un possible encrassement, "biofouling" ou saturation de la surface des électrodes avec des protéines, des lipides ou des cellules.

Chaque électrode 14, 15 peut être réalisée en deux parties conductrices distinctes :
- Une première partie agencée sur la surface interne d'une paroi du flacon 1, par exemple la paroi inférieure 10, dans le volume interne du flacon, pour être en contact avec l'échantillon liquide à analyser, placé dans le flacon ;
- Une deuxième partie reliée à la première partie à travers une paroi du flacon, par exemple la paroi inférieure 10, et agencée sur la surface externe du flacon de manière à former un contact électrique 141, 151 à connecter ;

La première partie de chaque électrode peut être réalisée sous la forme d'une pastille 140, 150 (figure 1B). La pastille peut être logée dans une cavité de la paroi inférieure 10 et être affleurante par rapport à la surface interne du flacon.

Les deux pastilles peuvent être chacune réalisées sous la forme d'un dépôt d'une encre conductrice sérigraphiée sur la surface interne de la paroi inférieure.

La première partie de l'électrode de mesure 14 peut être réalisée dans un matériau choisi parmi l'oxyde d'iridium qui est un oxyde dont le potentiel redox dépend du pH et un polymère conducteur, tel que la polyaniline (PAni).

La première partie de l'électrode de référence 15 peut être du type Ag/AgCI, classiquement utilisé en électrochimie.

Les deux contacts électriques 141, 151 du flacon sont intégrés à ladite première paroi de manière à former chacun un plot de contact. Une variante de réalisation des contacts électriques sera décrite ci-après.

Le flacon 1 peut porter une étiquette d'identification, par exemple de type RFID, dans laquelle sont stockées les données liées au prélèvement effectué (nom du patient, quantité prélevée, date et heure du prélèvement...). On verra que cette étiquette peut être lue par l'unité de traitement UC du système et ses données mémorisées par l'unité de traitement. L'unité de traitement UC pourra ensuite corréler directement les résultats du test aux données du prélèvement préalablement mémorisées.

En plus du flacon 1 ainsi équipé, le système comporte un caisson 2 destiné à recevoir le flacon pour analyse.

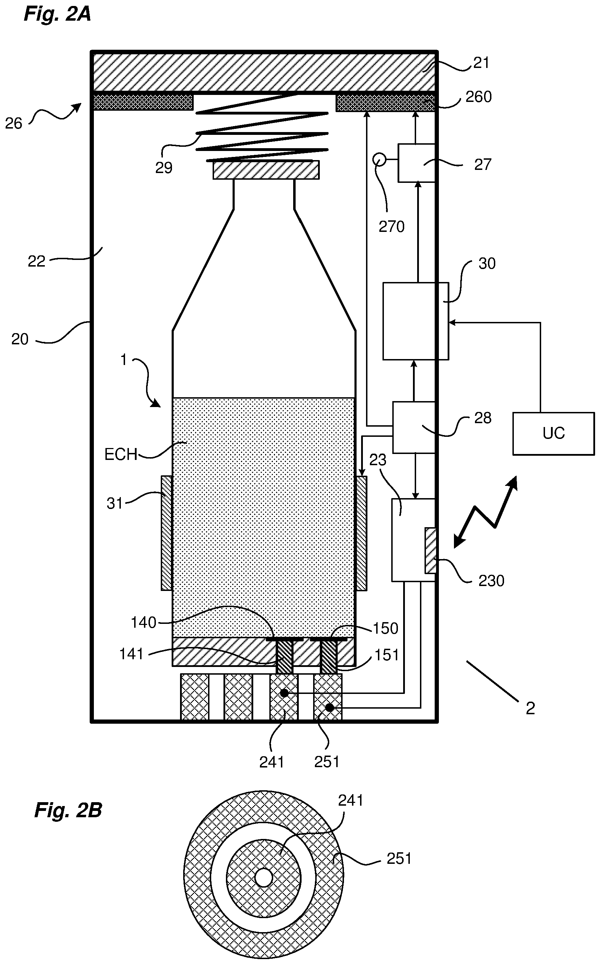

En référence à la figure 2A, le caisson 2 peut être composé d'un réceptacle 20 et d'un couvercle 21 destiné à être apposé sur le réceptacle 20 pour fermer le caisson de manière hermétique. Le caisson présente des propriétés d'isolation thermique permettant d'isoler thermiquement son espace interne par rapport à l'extérieur. Il peut s'agir d'une solution à double-paroi.

Le caisson 2 comporte ainsi un espace interne 22 délimité par les parois de son réceptacle 20 et de son couvercle 21.

Le système comporte également :
- Une unité électronique d'acquisition 23 de données de mesures de pH qui est intégrée audit caisson,
- Deux autres contacts électriques 241, 251 reliées électriquement à l'unité électronique d'acquisition 23 et agencés à l'intérieur du caisson 2,
- Une unité de chauffage 26 intégrée au caisson pour chauffer l'espace interne du caisson,
- Une unité de mesure et de gestion de la température 27 comprenant notamment une sonde 270 de mesure de température placée dans l'espace interne 22 du caisson 2,
- Une source d'alimentation électrique 28 pour alimenter l'unité électronique d'acquisition 23 et l'unité de chauffage 26.

L'unité d'acquisition 23 peut être une carte électronique classique configurée pour mesurer le potentiel électrique entre les deux électrodes 14, 15 du flacon 1. Les données de mesure sont enregistrées dans la carte. La carte peut comporter un module de communication 230. Le module de communication 230 peut être de type sans-fil et fonctionner sous un protocole tel que Bluetooth ou équivalent.

Comme représenté sur la figure 2B, les deux contacts électriques 241, 251 du caisson 2 peuvent être réalisés sous la forme de deux anneaux métalliques conducteurs agencés de manière concentrique. Ils sont avantageusement positionnés à l'intérieur du caisson, au fond de celui-ci et de manière adaptée pour connecter les deux plots de contact du flacon 1 lorsque ce dernier est inséré à l'intérieur du caisson. La réalisation des deux contacts en anneau permet de venir connecter le flacon 1 à 360° autour de son axe lorsqu'il est inséré dans le caisson 2.

En alternative, les deux contacts en anneaux concentriques pourraient être portés par le flacon 1 et les plots de contact par le caisson 2. Il serait également possible de prévoir des contacts électriques en anneaux concentriques à la fois sur le flacon 1 et sur le caisson 2.

Le flacon 1 est destiné à être placé dans l'espace interne du caisson. Le caisson 2 et le flacon 1 peuvent coopérer entre eux pour assurer une pression de contact entre les contacts électriques 141, 151 du flacon et les contacts électriques 241, 251 du caisson. Il peut s'agir d'un ressort 29 fixé au couvercle 21 et venant prendre appui contre le flacon 1 lorsque le couvercle 21 est fermé sur le réceptacle 20, pour exercer une pression du flacon contre le fond du caisson 2. Toute autre solution peut bien entendu être envisagée.

L'unité de chauffage 26 est de type électrique et peut comporter une résistance chauffante placée dans l'espace interne du caisson ou une solution de type module à effet Peltier. Le chauffage devra être réalisé de manière homogène, tout autour du flacon. De manière non limitative, sur la figure 2A, la résistance 260 est positionnée sous le couvercle 21. Le contact entre la résistance et la source d'alimentation électrique 28 peut être établi lorsque le couvercle est 21 est fermé sur le réceptacle 20. Un mécanisme de contacts glissants peut être prévu.

L'unité de mesure et de gestion de la température 27 comporte une sonde de température 270 et des moyens de régulation de ladite température à une valeur fixe déterminée. La valeur de température est choisie en fonction du type de culture mise en oeuvre. Dans le cas d'une hémoculture, la température d'incubation est choisie égale à 35°C +/- 2°C.

En dehors du sang, le test de stérilité peut être réalisé pour d'autres échantillons liquides. Il est en effet envisageable de prélever dans le flacon d'autres liquides biologiques, par exemple des liquides articulaires, pleuraux, péricardiques et autres liquides de ponction, liquides vitrés et des pus. La température d'incubation sera également de 35°C +/- 2°C. Pour les tests de stérilité la température d'incubation est de 30-35°C pour le milieu thioglycolate et de 20-25°C pour le milieu caséine-soja.

L'unité de mesure et de gestion de la température 27 peut comporter un thermostat destiné à recevoir la valeur de température mesurée et à commander l'unité de chauffage 26 pour réguler la température à la valeur souhaitée.

Le système peut également comporter une interface homme-machine 30. Cette interface peut être composée d'un écran agencé sur le boîtier du caisson 2 et d'organes de commande, destinés notamment à régler la température de chauffage à l'intérieur du caisson. Elle peut également comporter des indicateurs lumineux renseignant sur l'état de fonctionnement du système et sur l'état de la détection (allumage d'un voyant en cas de détection de présence de bactéries).

La source d'alimentation électrique 28 peut être constituée d'une batterie rechargeable logée dans un compartiment du caisson. Elle est destinée à alimenter l'unité de chauffage 26, l'unité de mesure et de régulation de température 27, l'unité d'acquisition de données de mesure 23 et l'interface homme-machine 30 si cette dernière est présente.

Comme déjà précisé ci-dessus, le système comporte une unité de traitement UC comportant un microprocesseur et des moyens de mémorisation. Elle peut également comporter un module de communication. Ce module peut être sans-fil pour communiquer via une liaison sans-fil, par exemple selon le protocole Bluetooth, avec le module 230 correspondant de l'unité d'acquisition 23.

L'unité de traitement UC est configurée pour :
- Recevoir les données de mesure en provenance de l'unité d'acquisition 23, par exemple via la liaison sans-fil,
- Etablir la courbe d'évolution du pH du mélange en fonction du temps,
- Traiter la courbe obtenue et déterminer la présence de micro-organismes.
- Commander l'interface homme-machine 30 fixée au caisson pour indiquer la présence de bactéries,
- Lire l'étiquette RFID présente sur le flacon 1 en vue de récolter et de stocker les données liées au prélèvement,
- Assurer la régulation de l'unité de chauffage 26 pour assurer une température d'incubation la plus constante possible.

L'unité de traitement UC peut être constituée d'un ordinateur personnel externe au caisson 2 ou être directement intégrée au caisson 2, permettant ainsi de disposer d'un outil monobloc et autonome en énergie et en fonctionnement. Dans ce dernier cas, elle serait alimentée par la source d'alimentation 28 du caisson.

Le système peut comporter une unité d'agitation 31 du flacon 1 qui est placée dans le caisson. Cette unité d'agitation est intégrée au caisson et peut notamment permettre de favoriser le mélange entre l'échantillon liquide prélevé et placé dans le flacon et le milieu de culture ajouté dans le flacon 1 pour la croissance des micro-organismes. Cette unité d'agitation peut être constituée d'un plateau ou manchon vibratoire (comme sur la figure 2A) intégré au caisson 2, et sur lequel vient prendre appui le flacon ou de toute autre solution équivalente. Cette unité d'agitation peut être également un aimant rotatif (aimant posé sur un moteur), de manière à faire tourner un barreau aimanté jetable placé dans l'échantillon (agitation magnétique). L'unité d'agitation 31 est avantageusement alimentée par la source d'alimentation 28.

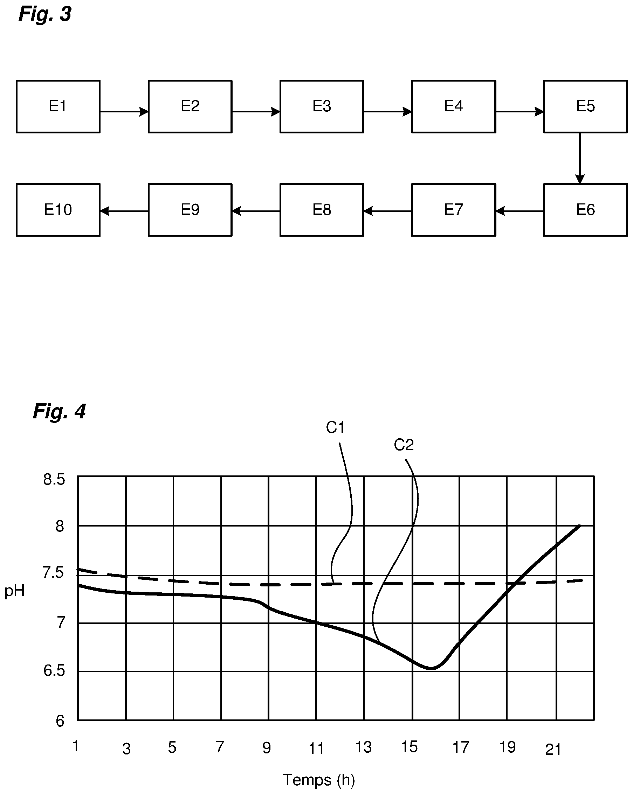

En référence à la figure 3, après prélèvement d'un échantillon sur un patient, par exemple en vue d'un test de stérilité, le principe de fonctionnement du système peut notamment être le suivant :
- E1 : Introduction de l'échantillon liquide prélevé dans le flacon 1 contenant déjà un milieu de culture adapté à la culture de micro-organismes.
- E2 : Optionnellement, lecture de l'étiquette RFID par l'unité de traitement UC en vue de récolter les données liées au prélèvement effectué.
- E3 : Introduction du flacon 1 ainsi rempli dans le réceptacle 20 et fermeture du couvercle 21 en vue d'assurer le contact électrique grâce au ressort 29. Des butées et mécanismes de détrompage peuvent permettre de caler le flacon 1 dans le réceptacle 20 et assurer la liaison électrique entre les contacts électriques 141, 151 du flacon et les contacts électriques 241, 251 du caisson.
- E4 : Réglage de la température dans l'espace interne du caisson à une valeur fixe adaptée au milieu de culture. Le réglage peut être réalisé directement via l'interface homme-machine 30 intégrée au caisson 2. Le chauffage du flacon doit être réalisé de manière homogène à une température fixe et régulée, pour favoriser la croissance des éléments pathogènes et leur détection. En outre, il peut être également possible de régler la durée d'incubation. Classiquement, celle-ci dure 5 jours mais peut parfois être prolongée à 15 jours dans certaines situations (par exemple : suspicion d'endocardite) ou pour certains prélèvements (par exemple : liquides articulaires). Les tests de stérilité durent en général plus longtemps (jusqu'à 3 semaines d'incubation avant de pouvoir délivrer un résultat négatif) car les micro-organismes contaminants peuvent être fastidieux et/ou très stressés.
- E5 : Avantageusement, activation de l'unité d'agitation 31 pour favoriser le mélange entre l'échantillon liquide et le milieu de culture. Elle raccourcit également le temps de détection des micro-organismes en assurant l'homogénéisation du milieu de culture pendant l'incubation, ainsi que l'oxygénation de ce milieu.
- E6 : Mesure du pH dans le mélange liquide présent dans le flacon 1 et acquisition des données de mesure de pH au cours du temps par l'unité d'acquisition 23.
- E7 : Transfert des données de mesure obtenues par l'unité d'acquisition 23 vers l'unité de traitement UC, par exemple via la liaison de communication sans-fil.
- E8 : Etablissement par l'unité de traitement UC d'une courbe de variation de pH en fonction du temps.
- E9 : Analyse de la courbe obtenue par l'unité de traitement UC et détermination de la présence ou de l'absence de micro-organismes par analyse de ladite courbe de variation du pH en fonction du temps.
- E10 : En cas de détection de présence de bactérie, commande de l'interface homme-machine 30 par l'unité de traitement 23 pour signaler cet état.

A titre d'exemple, la figure 4 montre deux courbes de variation du pH obtenues lors d'une hémoculture mise en oeuvre grâce au système de l'invention. La première courbe C1 a été obtenue pour un échantillon témoin, sans bactérie et la deuxième courbe C2 obtenue pour un échantillon comportant des bactéries (à une concentration initiale de 10 cfu/ml).

Sur la courbe C2, on peut notamment distinguer une variation à la baisse du pH après 8 h, synonyme de la présence de bactéries dans l'échantillon analysé.

La détection de la variation de pH peut être réalisée par exemple à l'aide d'un seuil appliqué à la dérivée première de la courbe de pH. Bien entendu, toute autre solution de traitement de la courbe pourrait être envisagée. Dans tous les cas, il s'agit de détecter une baisse du pH, synonyme d'une augmentation d'acidité du milieu aqueux suite à l'émission de CO₂ par les micro-organismes.

Afin de fiabiliser la mesure, il serait possible d'intégrer plusieurs capteurs pH dans le système.

## Revendications

1. Système de détection de présence de micro-organismes présent dans un échantillon, **caractérisé en ce qu'**il comporte :
- Un caisson (2) présentant un espace interne (22) fermé délimité par un réceptacle (20) et un couvercle (21) destiné à être apposé sur le réceptacle pour fermer le caisson de manière hermétique,
- Une unité électronique d'acquisition (23) de données de mesures de pH qui est intégrée audit caisson (2),
- Deux deuxièmes contacts électriques (241, 251) reliés à l'unité électronique d'acquisition (23) et agencés à l'intérieur du caisson (2),
- Une unité de chauffage (26) intégrée au caisson pour chauffer l'espace interne (22) du caisson,
- Une unité de mesure et de gestion de la température (27) comprenant une sonde (270) de mesure de température placée à l'intérieur du caisson,
- Une source d'alimentation électrique (28) pour alimenter l'unité électronique d'acquisition (23) et l'unité de chauffage (26),
- Une unité de traitement (UC) reliée à ladite unité électronique d'acquisition (23) et configurée pour traiter les données de mesure et comportant un module de détection de présence de micro-organismes par analyse de la variation du pH mesuré dans l'échantillon liquide,
- Un conteneur instrumenté logé dans l'espace interne du réceptacle et destiné à recevoir ledit échantillon, ledit conteneur comprenant :
- Des parois comportant une première surface dite interne, en contact avec son volume interne destiné à recevoir ledit échantillon et une surface dite externe opposée à ladite surface interne,
- Une électrode de référence (15) et une électrode de mesure (14) de pH intégrées à au moins une première paroi (10) desdites parois du conteneur et comprenant chacune au moins deux parties conductrices reliées entre elles, une première partie agencée sur la surface interne et destinée à être en contact avec l'échantillon liquide placé à l'intérieur du conteneur et une deuxième partie agencée sur la surface externe et comprenant deux premiers contacts électriques (141, 151) distincts accessibles à l'extérieur du conteneur, ses premiers contacts électriques (141, 151) étant en appui chacun contre un deuxième contact électrique (241, 251) distinct dudit caisson (2).

2. Système selon la revendication 1, **caractérisé en ce que** les deuxièmes contacts électriques (241, 251) sont réalisés sous la forme de deux anneaux conducteurs agencés de manière concentrique.

3. Système selon la revendication 1 ou 2, **caractérisé en ce que** l'unité électronique d'acquisition (23) comporte un premier module de communication sans-fil (230) et **en ce que** l'unité de traitement (UC) comporte un deuxième module de communication sans-fil relié au premier module de communication sans-fil via une liaison de communication sans-fil.

4. Système selon l'une des revendications 1 à 3, **caractérisé en ce qu'**il comporte une unité d'agitation par vibration intégrée audit réceptacle (20).

5. Système selon l'une des revendications 1 à 4, **caractérisé en ce que** l'unité de traitement (UC) est intégrée audit caisson (2).

6. Système selon l'une des revendications 1 à 5, **caractérisé en ce que** la première partie de l'électrode de référence (15) et celle de l'électrode de mesure (14) sont réalisées chacune sous la forme d'une pastille (140, 150) affleurante à la surface interne de ladite première paroi.

7. Système selon la revendication 6, **caractérisé en ce que** les deux pastilles (140, 150) sont réalisées chacune sous la forme d'un dépôt d'une encre conductrice sérigraphiée sur la surface interne de ladite première paroi.

8. Système selon la revendication 7, **caractérisé en ce que** la pastille (140) de l'électrode de mesure (14) est réalisée dans un matériau choisi parmi l'oxyde d'iridium et un polymère conducteur.

9. Système selon la revendication 7 ou 8, **caractérisé en ce que** la pastille (150) de l'électrode de référence est de type Ag/AgCI.

10. Système selon l'une des revendications 1 à 9, **caractérisé en ce que** les deux premiers contacts électriques (141, 151) sont intégrés à ladite première paroi de manière à former chacun un plot de contact ou deux anneaux concentriques.

11. Procédé de détection de présence micro-organismes dans un échantillon liquide mis en oeuvre à l'aide du système tel que défini dans l'une des revendications 1 à 10, **caractérisé en ce qu'**il comporte les étapes suivantes : Introduction de l'échantillon liquide et d'un milieu de culture dans le conteneur (1) pour obtenir un mélange liquide,
- Introduction dudit conteneur (1) dans l'espace interne (22) du caisson,
- Réglage de la température d'incubation dans l'espace interne du caisson (2) à une valeur fixe adaptée au milieu de culture,
- Mesure du pH au cours du temps dans le mélange liquide présent dans le conteneur (1) pour obtenir une courbe de variation du pH en fonction du temps,
- Détermination de la présence ou de l'absence de micro-organismes par analyse de ladite courbe de variation du pH en fonction du temps.

12. Procédé selon la revendication 11, **caractérisé en ce qu'**il comporte une étape d'agitation du conteneur pour mélanger l'échantillon liquide et le milieu de culture.

13. Procédé selon la revendication 11 ou 12, **caractérisé en ce qu'**il comporte une étape de réglage de la durée d'incubation.

## Patentansprüche

1. System zum Nachweis des Vorhandenseins von in einer Probe vorliegenden Mikroorganismen, **dadurch gekennzeichnet, dass** es Folgendes umfasst:
- ein Gehäuse (2) mit einem abgeschlossenen Innenraum (22), der von einem Behälter (20) und einem Deckel (21) begrenzt wird, der dafür ausgelegt ist, auf dem Behälter angebracht zu werden, um das Gehäuse hermetisch zu verschließen,
- eine elektronische Erfassungseinheit (23) für pH-Messdaten, die in das Gehäuse (2) integriert ist,
- zwei zweite elektrische Kontakte (241, 251), die mit der elektronischen Erfassungseinheit (23) verbunden und im Inneren des Gehäuses (2) angeordnet sind,
- eine in das Gehäuse integrierte Heizeinheit (26) zum Erwärmen des Innenraums (22) des Gehäuses,
- eine im Inneren des Gehäuses platzierte Temperaturmess- und -steuerungseinheit (27), die eine Temperaturmesssonde (270) umfasst,
- eine elektrische Stromversorgungsquelle (28) zum Versorgen der elektronischen Erfassungseinheit (23) und der Heizeinheit (26),
- eine Verarbeitungseinheit (UC), die mit der elektronischen Erfassungseinheit (23) verbunden und dafür ausgelegt ist, die Messdaten zu verarbeiten, und die ein Modul zum Nachweis des Vorhandenseins von Mikroorganismen durch Analyse der Änderung des in der flüssigen Probe gemessenen pH-Werts umfasst, und
- ein instrumentiertes Behältnis, das im Innenraum des Behälters untergebracht und dafür ausgelegt ist, die Probe aufzunehmen, wobei der Behälter Folgendes umfasst:
- Wände mit einer ersten, so genannten inneren Oberfläche in Kontakt mit seinem Innenvolumen, das dafür ausgelegt ist, die Probe aufzunehmen, und mit einer so genannten äußeren Oberfläche, die der inneren Oberfläche gegenüberliegt, und
- eine Referenzelektrode (15) und eine pH-Messelektrode (14), die in mindestens eine erste Wand (10) der Wände des Behältnisses integriert sind und jeweils mindestens zwei miteinander verbundene leitende Abschnitte umfassen, einen ersten Abschnitt, der auf der inneren Oberfläche angeordnet und dafür ausgelegt ist, mit der in das Innere des Behältnisses eingebrachten flüssigen Probe in Kontakt zu kommen, und einen zweiten Abschnitt, der auf der äußeren Oberfläche angeordnet ist und zwei von der Außenseite des Behältnisses zugängliche, separate erste elektrische Kontakte (141, 151) umfasst, wobei seine ersten elektrischen Kontakte (141, 151) jeweils an einem separaten zweiten elektrischen Kontakt (241, 251) des Gehäuses (2) anliegen.

2. System nach Anspruch 1, **dadurch gekennzeichnet, dass** die zweiten elektrischen Kontakte (241, 251) in Form zweier konzentrisch angeordneter leitfähiger Ringe ausgeführt sind.

3. System nach Anspruch 1 oder 2, **dadurch gekennzeichnet, dass** die elektronische Erfassungseinheit (23) ein erstes drahtloses Kommunikationsmodul (230) umfasst, und dadurch, dass die Verarbeitungseinheit (UC) ein zweites drahtloses Kommunikationsmodul umfasst, das mit dem ersten drahtlosen Kommunikationsmodul über eine drahtlose Kommunikationsverbindung verbunden ist.

4. System nach einem der Ansprüche 1 bis 3, **dadurch gekennzeichnet, dass** es eine in den Behälter (20) integrierte Vibrationsrührvorrichtung umfasst.

5. System nach einem der Ansprüche 1 bis 4, **dadurch gekennzeichnet, dass** die Verarbeitungseinheit (UC) in das Gehäuse (2) integriert ist.

6. System nach einem der Ansprüche 1 bis 5, **dadurch gekennzeichnet, dass** jeweils der erste Abschnitt der Referenzelektrode (15) und der Messelektrode (14) in Form eines Chips (140, 150) ausgeführt sind, der mit der inneren Oberfläche der ersten Wand bündig abschließt.

7. System nach Anspruch 6, **dadurch gekennzeichnet, dass** die beiden Chips (140, 150) jeweils in Form einer Deposition einer leitfähigen Tinte im Siebdruckverfahren auf der inneren Oberfläche der ersten Wand hergestellt sind.

8. System nach Anspruch 7, **dadurch gekennzeichnet, dass** der Chip (140) der Messelektrode (14) aus einem aus Iridiumoxid und einem leitfähigen Polymer ausgewählten Material hergestellt ist.

9. System nach Anspruch 7 oder 8, **dadurch gekennzeichnet, dass** der Chip (150) der Referenzelektrode vom Ag/AgCI-Typ ist.

10. System nach einem der Ansprüche 1 bis 9, **dadurch gekennzeichnet, dass** die beiden ersten elektrischen Kontakte (141, 151) so in die erste Wand integriert sind, dass sie jeweils eine Kontaktstelle oder zwei konzentrische Ringe bilden.

11. Verfahren zum Nachweis des Vorhandenseins von Mikroorganismen in einer flüssigen Probe, das mithilfe des Systems nach einem der Ansprüche 1 bis 10 durchgeführt wird, **dadurch gekennzeichnet, dass** es die folgenden Schritte umfasst: Einbringen der flüssigen Probe und eines Kulturmediums in das Behältnis (1), um ein flüssiges Gemisch zu erhalten,
- Einbringen des Behältnisses (1) in den Innenraum (22) des Gehäuses,
- Einstellen der Inkubationstemperatur im Innenraum des Gehäuses (2) auf einen an das Kulturmedium angepassten konstanten Wert,
- Messen des pH-Werts im Zeitverlauf in dem im Behältnis (1) vorliegenden flüssigen Gemisch, um eine Kurve der pH-Änderung in Abhängigkeit von der Zeit zu erhalten, und
- Bestimmung des Vorhandenseins oder Fehlens von Mikroorganismen durch Analyse der Kurve der pH-Änderung in Abhängigkeit von der Zeit.

12. Verfahren nach Anspruch 11, **dadurch gekennzeichnet, dass** es einen Schritt des Rührens des Behältnisses zum Mischen der flüssigen Probe und des Kulturmediums umfasst.

13. Verfahren nach Anspruch 11 oder 12, **dadurch gekennzeichnet, dass** es einen Schritt des Einstellens der Inkubationsdauer umfasst.

## Claims

1. System for detecting the presence of microorganisms present in a sample, **characterized in that** it comprises:
- an enclosure (2) comprising a closed internal space (22) bounded by a receptacle (20) and a lid (21) that is intended to be affixed to the receptacle to close the enclosure hermetically,
- an electronic unit (23) for acquiring pH-measurement data, which is integrated into said enclosure (2),
- two second electrical contacts (241, 251) that are connected to the electronic acquiring unit (23) and arranged inside the enclosure (2),
- a heating unit (26) integrated into the enclosure with a view to heating the internal space (22) of the enclosure,
- a unit (27) for measuring and managing temperature, comprising a temperature-measuring probe (270) placed inside the enclosure,
- an electrical power source (28) for powering the electronic acquiring unit (23) and the heating unit (26),
- a processing unit (UC) connected to said electronic acquiring unit (23) and configured to process the measurement data and comprising a module for detecting the presence of microorganisms via analysis of the variation in the pH measured in the liquid sample,
- an instrumented container housed in the internal space of the receptacle and intended to receive said sample, said container comprising:
- walls comprising a first surface, called the internal surface, which makes contact with the internal volume thereof intended to receive said sample, and a surface, called the external surface, opposite said internal surface,
- a reference electrode (15) and a pH-measuring electrode (14) that are integrated into at least a first wall (10) of said walls of the container and that each comprise at least two conductive portions that are connected together, a first portion arranged on the internal surface and intended to make contact with the liquid sample placed inside the container, and a second portion arranged on the external surface and comprising two separate first electrical contacts (141, 151) that are accessible from outside the container, its first electrical contacts (141, 151) each bearing against a separate second electrical contact (241, 251) of said enclosure (2).

2. System according to Claim 1, **characterized in that** the second electrical contacts (241, 251) take the form of two conductive rings arranged concentrically.

3. System according to Claim 1 or 2, **characterized in that** the electronic acquiring unit (23) comprises a first wireless communication module (230) and **in that** the processing unit (UC) comprises a second wireless communication module linked to the first wireless communication module via a wireless communication link.

4. System according to one of Claims 1 to 3, **characterized in that** it comprises a unit for agitating by vibration integrated into said receptacle (20).

5. System according to one of Claims 1 to 4, **characterized in that** the processing unit (UC) is integrated into said enclosure (2).

6. System according to one of Claims 1 to 5, **characterized in that** the first portion of the reference electrode (15) and the first portion of the measuring electrode (14) each take the form of a pad (140, 150) flush with the internal surface of said first wall.

7. System according to Claim 6, **characterized in that** the two pads (140, 150) each take the form of a deposit of a conductive ink screen-printed on the internal surface of said first wall.

8. System according to Claim 7, **characterized in that** the pad (140) of the measuring electrode (14) is made of a material chosen from iridium oxide and a conductive polymer.

9. System according to Claim 7 or 8, **characterized in that** the pad (150) of the reference electrode is of Ag/AgCI type.

10. System according to one of Claims 1 to 9, **characterized in that** the two first electrical contacts (141, 151) are integrated into said first wall so as to each form one contact land or two concentric rings.

11. Method for detecting the presence of microorganisms in a liquid sample, implemented using the system such as defined in one of Claims 1 to 10, **characterized in that** it comprises the following steps:
introducing the liquid sample and a culture medium into the container (1), to obtain a liquid mixture,
- introducing said container (1) into the internal space (22) of the enclosure,
- adjusting the incubation temperature in the internal space of the enclosure (2) to a set value suitable for the culture medium,
- measuring pH over time in the liquid mixture present in the container (1), to obtain a curve of pH variation as a function of time,
- determining the presence or absence of microorganisms via analysis of said curve of pH variation as a function of time.

12. Method according to Claim 11, **characterized in that** it comprises a step of agitating the container to mix the liquid sample and the culture medium.

13. Method according to Claim 11 or 12, **characterized in that** it comprises a step of adjusting the incubation time.
